Europäisches Patentamt

European Patent Office

Office européen des brevets

(19)

(11) Publication number: **0 150 960**
**B1**

(12) **EUROPEAN PATENT SPECIFICATION**

(45) Date of publication of patent specification: **03.01.90**

(51) Int. Cl.⁵: **A 61 M 25/00**

(21) Application number: **85300326.7**

(22) Date of filing: **17.01.85**

(60) Divisional application **89104838.1 filed on 17/01/85.**

(54) Catheter for retroinfusion of pharmacologic agents.

(30) Priority: **20.01.84 US 572411**

(43) Date of publication of application:
**07.08.85 Bulletin 85/32**

(45) Publication of the grant of the patent:
**03.01.90 Bulletin 90/01**

(84) Designated Contracting States:
**DE FR GB IT SE**

(56) References cited:
**WO-A-81/03613**
**DE-A-2 916 097**
**GB-A-2 060 131**
**US-A-2 687 131**
**US-A-3 509 884**
**US-A-4 328 056**
**US-A-4 363 321**

(73) Proprietor: **Corday, Eliot, Dr.**
**810 North Roxbury**
**Beverly Hills California 90210 (US)**

(72) Inventor: **Corday, Eliot**
**810 North Roxbury**
**Beverly Hills California 90210 (US)**
Inventor: **Meerbaum, Samuel**
**5741 El Canon**
**Woodland Hills California 91364 (US)**

(74) Representative: **Brown, John David et al**
**FORRESTER & BOEHMERT Widenmayerstrasse 4/I**
**D-8000 München 22 (DE)**

Courier Press, Leamington Spa, England.

EP 0 150 960 B1

## Description

BACKGROUND OF THE INVENTION
I. Field of the Invention

This invention relates generally to the use of catheters in the medical and veterinary arts and specifically to an inflatable balloon catheter for directed fluid retroinfusion, including a system of controlled retrograde administration of pharmacologic or contrast agents into the myocardium for their absorption so that they can be effectively utilized in the diagnosis or treatment of obstructive coronary artery disease.

II. Reference to the Prior Art

Cardiac retroperfusion consists of the delivery of oxygenated blood to jeopardized ischemic myocardium through a coronary venous vessel in a direction opposite to the normal flow of blood through that vessel and into the myocardial microcirculation when the normal arterial coronary channel through a myocardial region is obstructed. The concept of retroperfusion of the coronary veins as a surgical treatment for patients with coronary artery disease was introduced in the 1940's. (Beck, C.S.: Revascularization of the Heart, Surgery, Vol. 26, p. 82 1949.) The two-step Beck procedure consisted of creating a surgical shunt from the aorta (arterial blood) to the coronary sinus (venous circulation), and subsequently restricting the coronary sinus to facilitate retroperfusion of coronary veins with arterialized blood. However, long-term followup of these experiments demonstrated an unacceptable degree of myocardial and vascular damage. The unsatisfactory results reported in these followup studies were apparently due to the development of significant edema which caused damage to the coronary veins and myocardial tissue. This presumably resulted because of the chronic blockage of the coronary venous drainage causing a coronary venous pressure buildup which led to serious swelling of the heart tissue. (Beck, C.S., et al.: Operations for Coronary Disease, JAMA Vol. 13, pp. 1226-33 1954.) As a result of the considerable drawbacks and difficulties encountered, coupled with interest in forms of surgical revascularization of coronary arteries as a means for treating coronary artery disease, research in coronary sinus retroperfusion diminished. The concept of retroperfusion remains of limited interest in relation to management of patients exhibiting diffuse arteriosclerosis and poor distal coronary artery runoff, in which cases coronary bypass surgery is often not practicable.

In the 1960's research involving surgical retroperfusion was directed at development of a more regional coronary venous treatment for curtailing potential myocardial damage. In the early 1970's, research interest in retroperfusion turned toward a possible clinical method of treatment of the myocardium or preoperative and intraoperative support of heart function and viability in patients with coronary artery disease where other pharmacologic or standard circulatory assist interventions proved ineffective.

US-A-2687131 describes a female incontinence catheter having a balloon unit slidably mounted on a body portion and includes a central tubular portion, an expandable balloon which is preferably of pear shape and a rear tubular extension through which the inner end of an inflation tube passes into the space between the central tubular portion and the expandable balloon.

WO-A-81/03613 describes a multiple function catheter with multiple lumens including a transluminal gas pathway.

According to the present invention there is provided a catheter apparatus for retroinfusion of fluids into the coronary sinus for treatment or diagnosis of the myocardium comprising:

a hollow, flexible fluid delivery tube adapted to pass through the coronary sinus orifice and into the coronary sinus for delivery therethrough of pharmacologic fluids;

a balloon comprising elastic, foldable material adapted for inflation and secured and sealed proximally to the distal end of said hollow fluid delivery tube, said balloon having a broadened base and narrow tapered apex when inflated, the apex being towards the distal end, the base of said inflated balloon adapted to seal about the vein interfacing the coronary sinus orifice, and the apex portion of said inflated balloon tapering away from the walls of the vessel toward the center of the coronary sinus in order to avoid obstructing the middle cardiac vein to prevent pressure build up and edema and to facilitate drainage of the coronary sinus during cardiac systole;

means for delivery of fluid into said hollow fluid delivery tube;

means for securing said balloon to the distal extension of said fluid delivery tube; and

means for inflating said balloon.

The primary object of the present invention is to provide a coronary sinus catheter which permits directional coronary venous retroinfusion of fluids, (such as pharmacologic substances, namely, thrombolytic agents, antiarrhythmic and antiinflammatory drugs, and diagnostic contrast material) into the regionally diseased myocardium, applied for limited periods of time consistent with safe temporary interruption of coronary venous drainage, yet allowing adequate time for effective absorption and specific pharmaceutic performance of the administered agent. Another objective is to provide a coronary venous retroinfusion catheter with an improved anatomically-conforming inflated balloon configuration and material which will provide a unique shape designed for a more efficient seal against the walls of the coronary sinus without obstructing essential branches of the coronary veins which might tend to cause excessive pressure buildup in the coronary venous system. Other objects of the present invention will become apparent to one skilled in the art upon review of the more detailed description below.

The catheter according to this invention comprises a flexible hollow fluid delivery tube for passage therethrough of retroinfusate. The retroinfusate consists of various agents used in the treatment and diagnosis of the myocardium: (1) antiarrhythmic drugs such as lidocaine, procainamide, verapamil, propranolol, disopyramide and amiodarone; (2) coronary antispasmolytic drugs such as cardiazem, nifedipine, verapamil, dipyridamole, novocaine, and aminophyllin; (3) other cardioactive drugs including inotropes such as digitalis, catecholamines and mannitol; (4) thrombolytic agents such as streptokinase, tissue plasminogen activators; (5) antiinflammatory agents such as prostaglandins, steroids and glucose-insulin-potassium mixtures; and (6) various diagnostic contrast agents employed for angiography, radionuclide imaging or noniodinated echocardiographic imaging.

The proximal end of the hollow catheter tube is provided with a connecting means adapted for coupling thereto of distal pressure sensor adaptors, proximal syringes or other suitable types of perfusion or injection devices. The distal end of the hollow catheter fluid delivery tube is soft and rounded to prevent damage to the vessel valves and walls as it is winded through the branch or vessel to its ultimate destination in the coronary sinus. Just 1-4 cm proximal to the distal end of said hollow catheter tube is non-detachably affixed an inflatable balloon material structurally adapted to form an efficient seal against the walls of the coronary sinus when the catheter is inserted therethrough.

The catheter body further comprises a second hollow tube. In one embodiment said hollow tube can be used as a means for inflation of said balloon material. The distal extension of the inflating tube is located inside the cavity formed by the non-detachably secured balloon material just proximal to the distal end of the hollow delivery tube. In this embodiment the catheter body further comprises a third small lumen tube which is operatively connected to a pressure sensing device to provide pressure measurements in the coronary sinus. In another embodiment the distal extension of the hollow fluid delivery tube contains orifices located within the cavity formed by the non-detachably secured balloon material to allow for direct auto-inflation of the balloon material by the retroperfusate as the fluid is delivered through the tube and into the coronary sinus. In this embodiment the second hollow tube serves as a channel operatively connected to a pressure sensing means for constant monitoring of coronary venous pressures.

Upon insertion of the retroperfusion catheter through the coronary sinus orifice so that the balloon material can be deployed within the coronary sinus, the balloon is inflated for predetermined safe and effective periods allowing for retroinfusion and subsequent absorption of the retroinfusate by the distal myocardium. The balloon is subsequently deflated in order to allow appropriate periods of physiologic coronary venous drainage. The inflated balloon is an acorn configuration with a broadened base and a narrow tapered apex which provides an anatomically-conforming obstruction against the walls of the coronary sinus vein interfacing the coronary sinus orifice thereby insuring essentially unidirectional retrograde infusion into the coronary sinus allowing for absorption by the ischemic region.

The catheter apparatus according to this invention provides a coronary venous pressure feedback means controlled by electrocardiographic-synchronized intermittent coronary venous occlusion with alternating periods of coronary venous drainage. By way of example, after a period of retrograde retro-instillation and subsequent absorption and runoff behind the entrapped balloon, lasting from two seconds to ten minutes, this fluid entrapment will terminate promptly if the pressure in the monitored coronary vein suddenly rises above a set limit when the balloon is immediately rapidly deflated. The pressure feedback mechanism thereby facilitates the restoration of normal coronary venous drainage via the restoration of normal coronary venous drainage via the coronary sinus into the right atrium. As a result of the specially tapered configuration of the inflated balloon material, the middle cardiac vein is not obstructed despite its positioning just inside the orifice of the coronary sinus. The tapered apex of the inflated balloon thereby allows the middle cardiac vein to communicate with other coronary veins and prevent excessive regional coronary venous pressure buildup during retroinfusion. The uniquely shaped balloon catheter inserted into the appropriate coronary vein will then provide retroperfusion permitting as much absorption of specifically infused pharmacologic agents as possible during balloon inflation, while balloon deflation after the retroinfusion is designed to avoid complications of myocardial edema, which hampered successful clinical application of retroperfusion procedures developed in the 1950's.

Delivery of the pharmacologic agent into the retroperfusate can be accomplished by a gravity feeding device which delivers a predetermined quantity of a specified agent per unit of time into the hollow fluid delivery tube. The pharmacologic agent may also be introduced into the catheter system by way of a retroinfusion pump which delivers a controlled and predetermined quantity of retroinfusate into the fluid delivery tube. The pump output is controlled by a feedback means which is operatively connected to a pressure measuring means which constantly monitors the pressure in the coronary sinus. When the pressure in the coronary sinus exceeds safe parameters, the pressure measuring means triggers the feedback control which in turn terminates the flow of fluid from the retroinfusate pump.

Brief Description of the Drawings

FIG. 1 is a side elevational schematic view of an auto-inflatable balloon catheter with a pressure sensing lumen according to one embodiment of this invention, shown with the balloon in its deflated condition;

FIG. 2 is a side elevational view of the catheter according to this invention, shown with the balloon in its inflated configuration;

FIG. 3 is the posterior anatomic view of the heart showing the coronary sinus and its branches as it empties into the right atrium showing the anatomical region into which the catheter is inserted;

FIG. 4 is a perspective view of the anatomical position of the inflated balloon relative to the coronary sinus orifice and middle cardiac vein;

FIG. 5 is a schematic view of one embodiment of this invention which depicts an elevated gravity-feed infusion means in which the retroinfusate is delivered from a suitable sterile container via a cannula into the fluid delivery tube for ultimate delivery into the coronary sinus. Also shown is a pressure sensing means which monitors the pressure in the coronary sinus;

FIG. 6 shows a schematic view of a pump-controlled infusion apparatus which delivers the retroinfusate into the fluid delivery tube and which is controlled by feedback means which operate to terminate pump flow when the pressure sensor indicates excessive pressure in the coronary sinus; and

FIG. 7 is a side elevational view of the distal extension of the auto inflatable catheter according to one embodiment of this invention showing the orifices located in that portion of the fluid delivery tube surrounded by the balloon material.

Description of the Preferred Embodiments

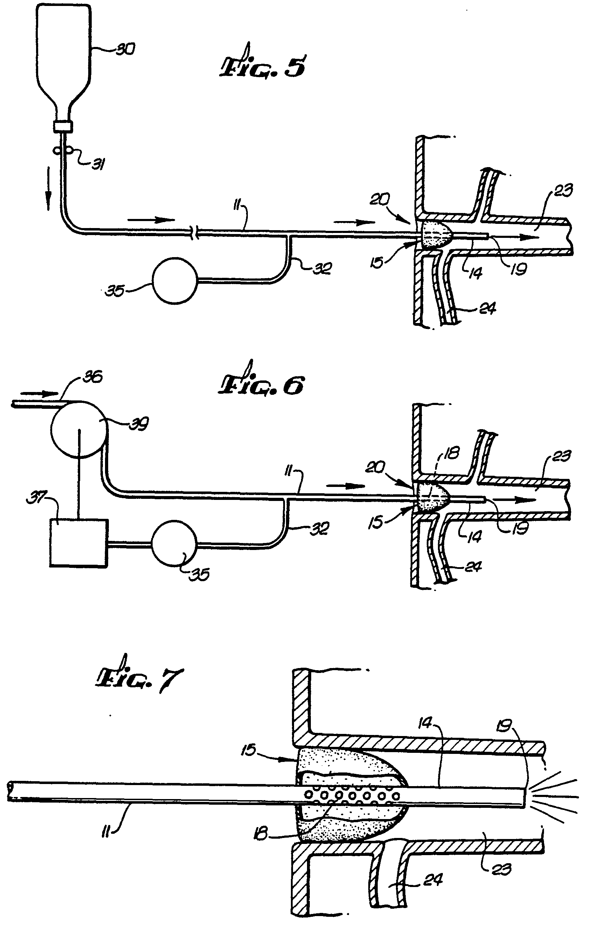

Referring to FIG. 1, the fluid delivery tube 11 of the catheter has at its proximal end standard connecting means adapted for coupling thereto of syringes or other suitable types of infusion devices. The hollow fluid delivery tube provides a channel for conveyance of retroperfusate (oxygenated arterial blood) or retroinfusate (diagnostic or pharmacologic agents for treatment of the myocardium) to the diagnostic or pathologic site in the myocardium. In order to gain access to the myocardium, the catheter must be inserted through a peripheral vessel (e.g., the jugular vein in the neck) and winded through that branch which leads to the right atrium and then inserted through the coronary sinus orifice into the coronary sinus communicating with the target area in the myocardium.

As a result of the delicate nature of the tissues in the vessels through which the catheter must pass, the catheter material must be flexible so as not to damage the valves or vessel walls of the branch into which it is inserted. It is particularly important that the distal extension of said hollow fluid delivery tube be soft and pliable since it is this portion of the tube which first comes into contact with the valves and other intimal structures of the vessel. A suitable elastomeric material provides these desirable properties and is ideal for the manufacture of the subject catheter body. The balloon material is elastic and maximally foldable to prevent tissue damage as it is winded through the vessel.

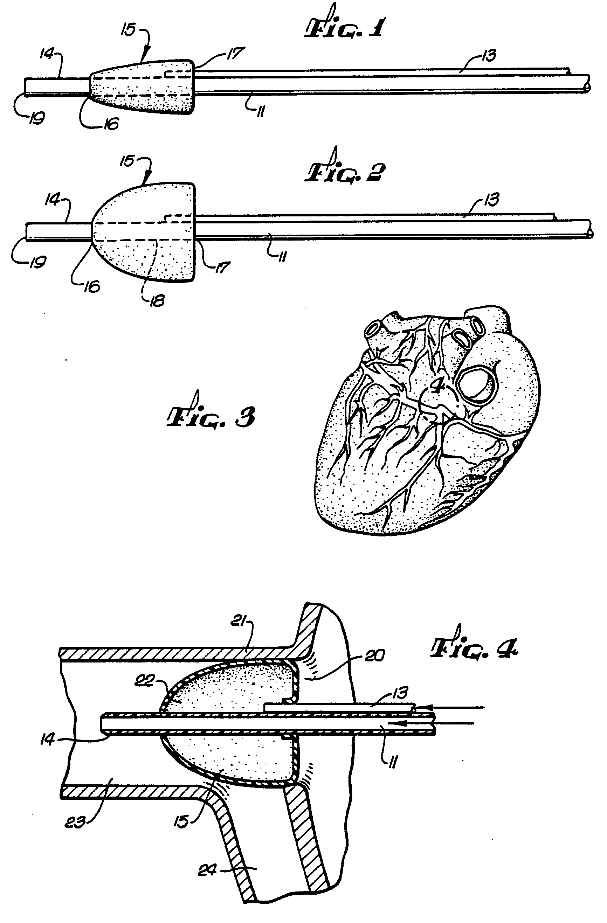

Once the fluid delivery tube is in proper position for instillation of retroinfusate in the coronary sinus, the balloon material 15 (shown deflated in FIG. 1 and inflated in FIG. 2) may be inflated to provide an anatomically conforming seal against the walls of the vein interfacing the coronary sinus orifice. The elastic material which comprises the inflatable balloon is non-detachably secured and sealed to the catheter body at points 16 and 17. In one embodiment, the balloon is inflated by the fluid which passes through the fluid delivery tube 11. As the fluid is passed through the fluid delivery tube 11, it enters the cavity formed by the flexible balloon material 15 through small orifices 18 located in that portion of the hollow fluid delivery tube which is surrounded by the balloon material. As the fluid enters the balloon cavity, the balloon is inflated. After the balloon is inflated, the fluid flow continues through the distal extension 14 of the fluid delivery tube and out of the distal orifice 19 and into the coronary sinus. In this embodiment, the small lumen hollow tube 13 serves as a channel through which the pressure in the coronary sinus can be measured when the tube is operatively attached to a pressure transducer.

In another embodiment, the small lumen hollow tube 13 provides a means for inflating the catheter balloon. In this embodiment, the hollow fluid delivery tube 11 contains no orifices in that portion of the hollow fluid delivery tube surrounded by the balloon material 15. The distal extension of the hollow inflation tube 13 is disposed within the cavity formed by the elastic balloon material which is permanently fused to secure and seal it to the catheter body.

FIG. 3 shows a posterior anatomic view of the heart depicting the coronary sinus and the venous branches which empty into the right atrium and the anatomical region through which the catheter is inserted at the coronary sinus orifice.

FIG. 4 shows a magnified view of the cutaway depicted in FIG. 3 showing the relative positions of the inflated balloon 15, distal extension of fluid delivery tube 14, coronary sinus 23, coronary sinus orifice 20 and middle cardiac vein 24. The balloon material when inflated by means of the hollow inflating tube 13 produces an acorn shaped balloon 15 which produces an anatomically-conforming seal against the vessel wall 21 and the coronary sinus orifice 20. The narrow apex 22 of said inflated acorn shaped balloon 15 tapers away toward the center of the coronary sinus 23 thereby preventing blockage of fluid entry into the middle cardiac vein 24. The shape of the inflated balloon thereby allows the middle cardiac vein to act as a pressure relief valve when the pressure within the coronary sinus increases, thereby preventing excessive pressure buildup and consequent edema.

FIG. 5 shows the auto-inflatable embodiment of the subject retroinfusion catheter in its inflated configuration just inside the coronary sinus orifice. Fluid delivery is facilitated by a gravity feeding infusion container 30 which dispenses a predetermined quantity of pharmacologic agent into the hollow fluid delivery tube 11. The quantity of retroinfusate delivered can be controlled by flow regulator 31 located at the base of the infusion apparatus. The balloon material 15 is inflated in the auto-inflatable embodiment by pressurized fluid flow through orifices 18 located in the distal extension of the fluid delivery tube in that portion of the tube surrounded by the balloon material 15. The fluid which comprises the retroinfusate flows through the channels formed by the orifices and into the cavity formed by the balloon material as it surrounds the hollow fluid delivery tube. The balloon is inflated just inside the coronary sinus orifice 20 to form a configuration with a broadened base and a narrow tapered apex to allow for the formation of a seal against the vein interfacing the coronary sinus orifice. The tapered apex of the balloon permits fluid flow through the middle cardiac vein 24 preventing thereby excessive pressure buildup and myocardial edema. After the balloon is inflated in diastole the fluid continues to flow through the distal extension 14 of the hollow fluid delivery tube and out of the distal orifice 19 and into the coronary sinus 23 for ultimate distribution and absorption by the pathological situs of the myocardium. A second small lumen tube 32 provides a means for communication of the fluid pressure from the fluid delivery tube 11 to a pressure sensing device 35 for constant monitoring of coronary venous pressure.

FIG. 6 shows the auto-inflatable embodiment of the retroinfusion catheter wherein the fluid is fed through a hollow tube 36 to a synchronous gas-actuated bladder pump 39 and into the hollow fluid delivery tube 11 for ultimate delivery into the coronary sinus 23. In this embodiment the small lumen tube 32 which provides a channel for communication of pressure changes in the coronary sinus is operatively attached to a pressure sensing device 35 which operatively communicates with a negative feedback means 37 which when activated by excessive pressures in the coronary sinus 23 triggers a reduction or termination of pump 39 operation providing thereby an automatic system of retroinfusate regulation.

FIG. 7 shows a magnified depiction of the hollow fluid delivery tube 11 and the orifice 18 which provides channels for entry of fluid into the cavity formed by the balloon material 15 for inflation of the auto-inflatable embodiment of the retroinfusion catheter. The narrow tapered apex of the balloon prevents obstruction of the middle cardiac vein allowing thereby drainage of the coronary sinus 23 when the coronary venous pressure exceeds safe parameters.

When the catheter according to this invention is inserted through the coronary sinus orifice by way of a peripheral vessel, the catheter is capable of stopping the natural flow of blood in the vessel when the balloon is inflated in cardiac diastole, reversing the direction of the natural flow of blood by injecting through the hollow fluid delivery tube shunted oxygenated arterial blood in retrograde fashion through the coronary sinus making thereby a retroperfusion, providing a mechanism for release of excessive pressure in the coronary sinus by allowing for drainage of fluid when necessary through the middle cardiac vein, deflating said balloon material and allowing a resumption of the natural anterograde blood flow from the coronary sinus through the coronary sinus orifice into the right atrium.

## Claims

1. A catheter apparatus for retroinfusion of fluids into the coronary sinus for treatment or diagnosis of the myocardium comprising:
   a hollow, flexible fluid delivery tube adapted to pass through the coronary sinus orifice and into the coronary sinus for delivery therethrough of pharmacologic fluids;
   a balloon comprising elastic, foldable material adapted for inflation and secured and sealed proximally to the distal end of said hollow fluid delivery tube, said balloon having a broadened base and narrow tapered apex when inflated, the apex being towards the distal end, the base of said inflated balloon adapted to seal about the vein interfacing the coronary sinus orifice, and the apex portion of said inflated balloon tapering away from the walls of the vessel toward the center of the coronary sinus in order to avoid obstructing the middle cardiac vein to prevent pressure build up and edema and to facilitate drainage of the coronary sinus during cardiac systole;
   means for delivery of fluid into said hollow fluid delivery tube;
   means for securing said balloon to the distal extension of said fluid delivery tube; and
   means for inflating said balloon.

2. A catheter according to Claim 1, wherein said balloon material is secured and sealed 1 to 4 centimeters proximally to the distal extension of said hollow fluid delivery tube.

3. A catheter according to Claim 1 or 2, wherein said means for delivery of fluid into said hollow fluid delivery tube consists of a gravity-feed infusion container adapted for controlled delivery of fluids into said fluid delivery tube.

4. A catheter according to Claim 1 or 2, wherein said means for delivery of fluid consists of a medical infusion pump for controlled administration of specified quantities of pharmacologic fluids into said hollow fluid delivery tube.

5. A catheter according to Claim 1, wherein the balloon is secured to the distal extension of said catheter body, by a cement which binds the balloon material to the catheter fluid delivery tube, and by ligatures for reinforcement.

6. A catheter according to Claim 1, wherein the means for inflating said balloon material consists of small orifices located in the hollow fluid delivery tube and disposed in the area of the cavity formed by the balloon material when sealed and secured at the distal extension of said hollow fluid delivery tube, said orifices providing channels allowing for conveyance of fluid from said hollow fluid delivery tube into the cavity formed by said balloon material and facilitating thereby inflation of the catheter balloon by the infusing fluid.

7. A catheter according to Claim 1, wherein said means for inflating said balloon material consists of a second hollow small lumen tube structurally connected to said fluid delivery tube, the distal extension of which is disposed inside the cavity formed by the balloon material when secured and sealed to said hollow fluid delivery tube, said small lumen inflating tube thereby facilitating the conveyance of fluid into the balloon cavity thereby inflating said catheter balloon by the flow of pressurized infusing fluid.

8. A catheter according to any one of the preceding claims, further comprising:
means to monitor the myocardial beat and to administer fluids through the catheter in a pulsatile manner following each heart beat when the heart is in diastole.

9. A catheter according to Claim 8, wherein said means to monitor the myocardial beat consists of an electrocardiograph.

10. A catheter according to Claim 8, wherein said means to administer fluids in a pulsatile manner consists of a synchronous gas-actuated bladder pump disposed for intermittent infusion of fluids into the hollow fluid delivery tube in a synchronous manner when the heart is in diastole, and means for monitoring retroperfusion flow rate and blood pressure consisting of an electromagnetic flowmeter probe and a pressure transducer operatively attached to said fluid delivery tube and disposed for constant fluid pressure measurement, and automatic feedback means for termination or reduction of pump flow when said pressure means indicates internal pressure parameters have been exceeded.

11. A catheter according to Claim 8, wherein said means to administer fluids through said catheter in a synchronous pulsatile manner consists of a gravity-feed infusion container which is operatively attached to said hollow fluid catheter delivery tube to allow for direct and controlled infusion of a predetermined quantity of pharmacologic agent per unit time into said hollow fluid delivery tube, and an electromagnetic flowmeter probe and pressure transducer for constant measurement of retroperfusion flow rate and blood pressure.

**Patentansprüche**

1. Kathetervorrichtung für Rückinfusion von Flüssigkeiten in den Koronarsinus zur Behandlung oder Diagnose des Myokards, gekennzeichnet durch
einen hohlen, biegsamen Flüssigkeitszuführschlauch, der durch die Koronarsinusmündung und in den Koronarsinus führen kann für die Zuführung von pharmakologischen Flüssigkeiten dorthindurch;
einen Ballon aus elastischem, faltbarem Material, das aufgeblasen werden kann und proximal zum distalen Ende besagten hohlen Flüssigkeitszuführschlauches befestigt und abgedichtet ist, wobei besagter Ballon eine verbreiterte Grundfläche und eine schmale verjüngte Spitze aufweist, wenn er aufgeblasen ist, wobei die Spitze zum distalen Ende hin zeigt, die Grundfläche besagten ausgeblasenen Ballons, die um die Vene herum abdichten kann, an der Koronarsinusmündung anliegt und der Spitzenbereich besagten aufgeblasenen Ballons sich von den Wänden des Gefäßes zur Mitte des Koronarsinus hin verjüngt, um zu verhindern, daß die mittlere Herzvene verlegt wird, um einen Druckaufbau und Ödeme zu verhindern und die Drainage des Koronarsinus während der Systole zu erleichtern;
Mittel für die Zuführung von Flüssigkeit in besagten hohlen Flüssigkeitszuführschlauch;
Mittel zum Befestigen besagten Ballons am distalen Fortsatz besagten Flüssigkeitszuführschlauchs; und
Mittel zum Aufblasen besagten Ballons.

2. Katheter nach Anspruch 1, dadurch gekennzeichnet, daß besagtes Ballonmaterial 1 bis 4 cm proximal zum distalen Fortsatz besagten hohlen Flüssigkeitszuführschlauchs befestigt und abgedichtet ist.

3. Katheter nach Anspruch 1 oder 2, dadurch gekennzeichnet, daß besagte Mittel für die Zuführung von Flüssigkeit in besagten hohlen Flüssigkeitszuführschlauch aus einem Infusionsbehälter mit Gefällezuführung besteht, der für kontrollierte Zuführung von Flüssigkeiten in besagten Flüssigkeitszuführschlauch geeignet ist.

4. Katheter nach Anspruch 1 oder 2, dadurch gekennzeichnet, daß besagte Mittel für die Zuführung von Flüssigkeit aus einer medizinischen Infusionspumpe zur kontrollierten Verabreichung von festgesetzten Mengen pharmakologischer Flüssigkeiten in besagten hohlen Flüssigkeitszuführschlauch besteht.

5. Katheter nach Anspruch 1, dadurch gekennzeichnet, daß der Ballon am distalen Fortsatz besagten Katheterkörpers mit einem Kleber, der das Ballonmaterial mit dem Katheterflüssigkeitszuführschlauch verbindet, und mit Verstärkungsbändern befestigt ist.

6. Katheter nach Anspruch 1, dadurch gekennzeichnet, daß die Mittel zum Aufblasen besagten Ballonmaterials aus kleinen Öffnungen besteht, die im hohlen Flüssigkeitszuführschlauch untergebracht und im Bereich des Hohlraums, der vom Ballonmaterial gebildet wird, wenn dieses am distalen Fortsatz besagten hohlen Flüssigkeitszuführschlauchs abgedichtet und befestigt ist, angeordnet sind, wobei besagte Öffnungen Kanäle zur Verfügung stellen, die den Transport von Flüssigkeit aus besagtem hohlen Flüssigkeitszuführschlauch in den von besagtem Ballonmaterial gebildeten Hohlraum ermöglichen und dadurch das Aufblasen des Katheterballons durch die Infusionsflüssigkeit erleichtern.

7. Katheter nach Anspruch 1, dadurch gekennzeichnet, daß besagte Mittel zum Aufblasen besagten Ballonmaterials aus einem zweiten hohlen, konstruktiv mit besagtem Flüssigkeitszuführschlauch verbundenen Schlauch mit kleinem Lumen besteht, dessen distaler Fortsatz innerhalb des Hohlraums angeordnet ist, der vom Ballonmaterial gebildet wird, wenn dieses an besagtem hohlen Flüssigkeitszuführschlauch befestigt und abgedichtet ist, wobei besagter Aufblasschlauch mit kleinem Lumen dadurch den Transport von Flüssigkeit in den Ballonhohlraum erleichtert, wodurch besagter Katheterballon durch den Zufluß unter Druck stehender Infusionsflüssigkeit aufgeblasen wird.

8. Katheter nach einem der vorangehenden Ansprüche, weiter gekennzeichnet durch

Mittel zum Überwachen des Herzmuskelschlags und zum stoßweisen Zuführen von Flüssigkeiten durch den Katheter, folgend auf jeden Herzschlag, wenn das Herz in der Diastole ist.

9. Katheter nach Anspruch 8, dadurch gekennzeichnet, daß besagte Mittel zum Überwachen des Herzmuskelschlages aus einem EKG-Schreiber bestehen.

10. Katheter nach Anspruch 8, dadurch gekennzeichnet, daß besagte Mittel zum stoßweisen Zuführen von Flüssigkeiten aus einer synchronen gasbetätigten Blasenpumpe, angeordnet zur intermittierenden Infusion von Flüssigkeiten in den hohlen Flüssigkeitszuführschlauch in synchroner Weise, wenn das Herz in der Diastole ist, und Mitteln zum Überwachen der Rückperfusionsstromgeschwindigkeit und des Blutdrucks, die aus einer elektromagnetischen Durchflußmessersonde und einem Druckmeßwertwandler, operativ verbunden mit besagtem Flüssigkeitszuführschlauch und zur konstante Flüssigkeitsdruckmessung angeordnet und automatischen Rückkopplungsmitteln zur Beendigung oder Verringerung des Pumpstroms bestehen, wenn besagte Druckmittel anzeigen, daß interne Druckparameter überschritten worden sind.

11. Katheter nach Anspruch 8, dadurch gekennzeichnet, daß besagte Mittel zum synchronen stoßweisen Verabreichen von Flüssigkeiten durch besagten Katheter aus einem Infusionsbehälter mit Gefällezuführung, der operativ mit besagtem hohlen Flüssigkeitskatheterzuführschlauch verbunden ist, um direkte und kontrollierte Infusion einer vorbestimmten Menge von pharmakologischem Agens pro Zeiteinheit in besagten hohlen Flüssigkeitszuführschlauch zu ermöglichen, und einer elektromagnetischen Durchflußmessersonde und einem Druckmeßwertwandler zur konstanten Messung der Rückperfusionsstromgeschwindigkeit und des Blutdrucks bestehen.

## Revendications

1. Dispositif catheter pour rétroperfusion de fluides à l'intérieur du sinus coronarien pour traitement ou diagnostic du myocarde comprenant:

un tube creux d'apport de fluide, flexible, adapté pour passer par l'orifice du sinus coronarien et pénétrer dans le sinus coronarien pour y injecter des fluides pharmaceutiques;

un ballon composé d'un matériau élastique, pliable, adapté pour le gonflage et fixé de façon étanche très près de l'extrémité finale dudit tube creux d'apport de fluide, ledit ballon ayant une base élargie et un sommet étroit rétréci quand il est gonflé, le sommet étant dirigé vers l'extrémité finale, la base dudit ballon gonflé étant adaptée pour étancher la veine qui est reliée à l'orifice du sinus coronarien, et la partie sommet dudit ballon gonflé allant en rétrécissant depuis les parois du vaisseau vers le centre du sinus coronarien afin d'empêcher l'obstruction de la veine cardiaque moyenne, éviter l'accumulation de pression et l'oedème, et faciliter le drainage du sinus coronarien pendant la systole cardiaque;

des moyens pour apporter du fluide dans ledit tube creux d'apport de fluide;

des moyens pour fixer ledit ballon à l'extrémité finale dudit tube d'apport de fluide et;

des moyens pour gonfler ledit ballon.

2. Catheter selon la revendication 1, dans lequel ledit matériau de ballon est fixé de façon étanche à une distance d'environ 1 à 4 cm de l'extrémité finale dudit tube creux d'apport de fluide.

3. Catheter selon la revendication 1 ou 2, dans lequel lesdits moyens pour l'apport de fluide dans le tube creux d'apport de fluide comprennent un récipient de perfusion à alimentation par gravité adapté pour l'apport commandé de fluides dans ledit tube d'apport de fluide.

4. Catheter selon la revendication 1 ou 2, dans lequel lesdits moyens pour l'apport de fluide comprennent une pompe de perfusion médicale pour l'administration commandée de quantités spécifiées de fluides pharmaceutiques dans ledit tube creux d'apport de fluide.

5. Catheter selon la revendication 1, dans lequel le ballon est fixé à l'extrémité finale dudit corps de catheter, par un ciment qui relie le matériau de ballon au tube d'apport de fluide du catheter et par des ligatures de renforcement.

6. Catheter selon la revendication 1, dans lequel les moyens pour gonfler ledit matériau de ballon comprennent de petits orifices situés dans le tube creux d'apport de fluide et disposés dans la zone de la cavité formée par le matériau de ballon quand il est étanché et fixé à l'extrémité finale dudit tube creux d'apport de fluide, lesdits orifices fournissant des canaux permettant le transport du fluide depuis ledit tube creux d'apport de fluide jusque dans la cavité formée par ledit matériau de ballon et facilitant ainsi le gonflage du ballon du catheter par le fluide de la perfusion.

7. Catheter selon la revendication 1, dans lequel lesdits moyens pour gonfler ledit matériau de ballon comprennent un second tube creux à petit orifice en liaison structurelle avec le tube d'apport de fluide, dont l'extrémité finale se trouve à l'intérieur de la cavité formée par le matériau de ballon quand il est fixé et étanche audit tube creux d'apport de fluide, ledit tube de gonflage à petit orifice facilitant ainsi le passage du fluide dans la cavité du ballon gonflant ainsi ledit ballon de catheter par le flux du fluide comprimé de perfusion.

8. Catheter selon l'une quelconque des revendications précédentes, comprenant en outre:

des moyens pour surveiller le battement du myocarde et pour administrer des fluides à travers le catheter de manière pulsée en suivant chaque battement du coeur lorsque le coeur est en diastole.

9. Catheter selon la revendication 8, dans lequel lesdits moyens pour surveiller le battement du myocarde comprennent un électrocardiographe.

10. Catheter selon la revendication 8, dans lequel lesdits moyens pour administrer des fluides de manière pulsée comprennent une pompe synchrone à vessie actionnée par gaz, prévue pour une perfusion intermittente de fluides dans le tube creux d'apport de fluide de manière synchrone quand le coeur est en diastole, et des moyens pour surveiller le débit de rétroperfusion et la pression du sang comprenant une sonde de débitmètre électromagnétique et un capteur de pression fixés en position de service audit tube d'apport de fluide et prévus pour la mesure permanente de la pression du fluide, et des moyens de réaction automatiques pour l'arrêt ou la réduction du flux de la pompe quand lesdits moyens de pression indiquant que les paramètres de pression interne ont été dépassés.

11. Catheter selon la revendication 8, dans lequel lesdits moyens pour administrer des fluides à travers ledit catheter de manière pulsée synchrone comprennent un récipient de perfusion à alimentation par gravité qui est fixé en position de service audit tube creux d'apport de fluide du catheter pour permettre la perfusion directe et commandée d'une quantité prédéterminée d'agents pharmaceutiques par unité de temps dans ledit tube creux d'apport de fluide et une sonde de débitmètre électromagnétique et un capteur de pression pour la mesure permanente du débit de rétroperfusion et de la pression du sang.

Fig. 5

Fig. 6

Fig. 7

*FIG. 1*

*FIG. 2*

*FIG. 3*

*FIG. 4*